(19) Europäisches Patentamt
European Patent Office
Office européen des brevets

(11) Veröffentlichungsnummer: **0 111 073**
**B1**

(12) **EUROPÄISCHE PATENTSCHRIFT**

(45) Veröffentlichungstag der Patentschrift: **25.02.87**

(51) Int. Cl.⁴: **C 07 D 233/06**

(21) Anmeldenummer: **83109426.3**

(22) Anmeldetag: **22.09.83**

(54) **Verfahren zur Herstellung von 2-Imidazolinen.**

(30) Priorität: **02.10.82 DE 3236596**

(43) Veröffentlichungstag der Anmeldung: **20.06.84 Patentblatt 84/25**

(45) Bekanntmachung des Hinweises auf die Patenterteilung: **25.02.87 Patentblatt 87/9**

(84) Benannte Vertragsstaaten: **CH DE FR GB IT LI**

(56) Entgegenhaltungen:
**EP-A-0 012 371**
**EP-A-0 036 521**
**CH-A-435 301**
**DE-A-2 512 513**
**FR-A-2 121 106**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft, Carl-Bosch- Strasse 38, D-6700 Ludwigshafen (DE)**

(72) Erfinder: **Dockner, Toni, Dr., Grossgasse 6, D-6701 Meckenheim (DE)**
Erfinder: **Frank, Anton, Bannwasserstrasse 72, D-6700 Ludwigshafen (DE)**

**EP 0 111 073 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft ein neues Verfahren zur Herstellung von 2-Imidazolinen durch Umsetzung von 1,2-Diaminen mit Nitrilen in Gegenwart von Kupfersalzen als Katalysatoren.

In 2-Stellung substituierte 2-Imidazoline lassen sich z.B. nach dem in J. Chem. Soc. (1947), Seiten 497 bis 505 beschriebenen Verfahren dadurch herstellen, daß man Salze des Ethylendiamins mit Nitrilen bei 100 bis 260°C umsetzt. Bei diesem Verfahren sind die Ausbeuten unbefriedigend. In der japanischen Patentveröffentlichung 24 965/1964 bzw. der DS-A-2 512 513 ist angegeben, daß man die Kondensation von 1,2-Diamin mit Nitrilen mit Schwefel bzw. mit polysulfiden katalysieren kann. Bei diesen Verfahren ist die Schwefelwasserstoffbildung von Nachteil. Sie erfordert aufwendige Vorrichtungen und Maßnahmen für eine Absaugung und Vernichtung des giftigen und geruchsbelästigenden Schwefelwasserstoffs. Nach den Angaben der FR-A-2 121 106 verwendet man als Katalysator für die Kondensation von 1,2-Diaminen mit Nitrilen zur Herstellung von 2-Imidazolinen Cystein, Cystin oder deren Salze. Dieses Verfahren ist hinsichtlich Ausbeute und Reinheit der Verfahrensprodukte unbefriedigend. In der EP-A-36 521 wird ein Verfahren zur Herstellung von 2-Imidazolinen beschrieben, bei dem man N,N'-Diformyl-1,2-diamine bei 200 bis 350°C in Gegenwart eines Inertgases und von Zinkoxid oder einem Gemisch aus Zinkoxid und Aluminiumoxid in der Gasphase umsetzt. Das Arbeiten in der Gasphase ist technisch aufwendig, außerdem ist es für höhersiedende N,N'-Diformyl-1,2-diamine wenig geeignet, da man bei ihrer Überführung in die Gasphase Zersetzungsreaktionen nicht vermeiden kann. Bei dem in der EP-A-12 371 beschriebenen Verfahren zur Herstellung von 2-Imidazolinen durch Umsetzung von Diaminen mit Nitrilen oder Carbonylverbindungen in Gegenwart von Oxiden oder Phosphaten der Metalle der 3. oder 4. Gruppe des Periodischen Systems oder von Siliciumdioxid wird ebenfalls in der Gasphase gearbeitet. Auch dieses Verfahren hat die obengenannten technischen Nachteile der Gasphasenreaktion. Es macht auch eine aufwendige Regenerierung der Katalysatoren erforderlich.

Es wurde nun gefunden, daß man 2-Imidazoline der Formel (I)

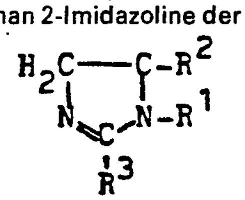 I,

in der $R^1$ und $R^2$ ein Wasserstoffatom, einen Alkylrest mit 1 bis 18 C-Atomen, einen Cyclohexyl- oder Cyclopentylrest, einen Aralkylrest mit 7 bis 12 C-Atomen oder einen Phenyl-oder Naphthylrest, der durch Alkyl- oder Alkoxygruppen mit jeweils 1 bis 4 C-Atomen substituiert sein kann, $R^3$ einen Alkylrest mit 1 bis 18 C-Atomen, einen Cyclohexyl- oder Cyclopentylrest, einen Aralkylrest mit 7 bis 12 C-Atomen oder einen Phenyl- oder Naphthylrest, der durch $C_1$-$C_4$ Alkyl- oder $C_1$-$C_4$ Alkoxygruppen substituiert sein kann, oder einen Rest der Formel

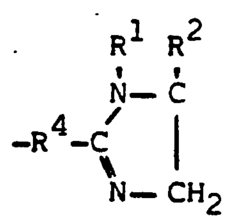

und $R^4$ einen Alkylenrest mit 1 bis 4 C-Atomen bedeuten, durch Umsetzung von 1,2-Diaminen der Formel (II)

$H_2C$ - CH - $R^2$> II

$H_2N$ NH-$R^1$

mit Nitrilen der Formel (III)

$R^5$ - CN III,

in der $R^5$ die für $R^3$ genannte Beteutung hat oder für den Rest -$R^4$-CN steht, erheblich vorteilhafter herstellen kann, wenn man die Umsetzung in der Flüssigphase bei Temperaturen von 110 bis 300°C und in Gegenwart von 0,1 bis 5 Gewichtsteilen, bezogen auf 100 Gewichtsteile Diamin, an Kupfersalzen vornimmt.

Die Umsetzung kann für den Fall der Verwendung von Ethylendiamin und Acetonitril bzw. Adipinsäuredinitril durch die folgenden Formeln wiedergegeben werden:

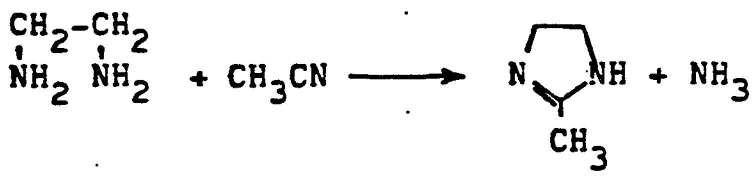

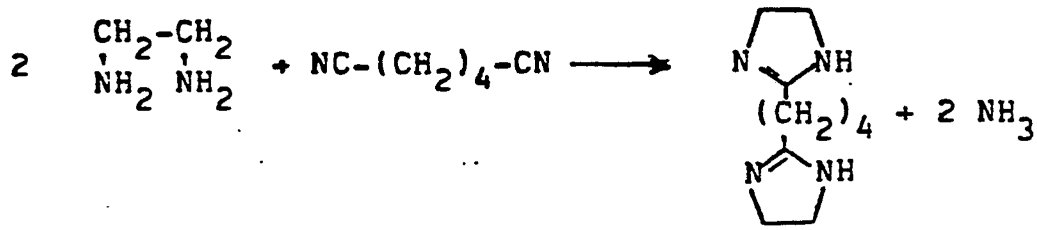

Im Vergleich zu den bekannten Verfahren liefert das Verfahren nach der Erfindung überraschend auf einfacherem und wirtschaftlicherem Wege 2-Imidazoline in höherer Ausbeute und Reinheit.

In den Ausgangsstoffen der Formel (II) stehen $R^1$ und $R^2$ z.B. für ein Wasserstoffatom. Die Substituenten $R^1$, $R^2$ können auch wie der Rest $R^3$ Alkylgruppen mit 1 bis 18, vorzugsweise 1 bis 7 C-Atomen, Cyclohexyl- oder Cyclopentylgruppen, Aralkylgruppen mit 7 bis 12 C-Atomen oder Phenyl- oder Naphthylgruppen, die durch $C_1$-$C_4$ Alkyl- oder $C_1$-$C_4$ Alkoxygruppen substituiert sein können, bedeuten. $R^4$ ist ein Alkylenrest mit 1 bis 4 C-Atomen.

Als Diamine der Formel (II) kommen z.B. in Frage: Ethylendiamin, 1,2-Propylendiamin, 1,2-Butylendiamin, 1,2-Pentylendiamin, 1,2-n-Hexylendiamin, 1,2-n-Heptylendiamin, 1,2-n-Octylendiamin, 1,2-n-Nonylendiamin, 1,2-n-Decylendiamin; 2-Cyclohexyl-, 2-Cyclopentyl-, 2-Benzyl-, 2-Phenyl-, o-Methoxyphenyl-, m-Methoxyphenyl:, p-Methoxyphenyl-, 2,5-Dimethylphenyl-, 2,6-Dimethylphenyl-, 2,4-Dimethylphenyl-, 2,3-Dimethylphenyl-, 2-o-Toluyl-, 2-m-Toluyl-, 2-p-Toluyl-, 2-o-Ethylphenyl-, 2-m-Ethylphenyl oder 2-p-Ethylphenyl-1,2-ethylendiamin; N-Cyclohexyl-, N-Cyclopentyl-, N-Benzyl-, N-Phenyl-, N-o-Methoxyphenyl-, N-m-Methoxyphenyl-, N-p-Methoxyphenyl-, N-2,5-Dimethylphenyl-, N-2,6-Dimethylphenyl-, N-2,4-Dimethylphenyl-, N-2,3-Dimethylphenyl-, N-o-Toluyl-, N-m-Toluyl-, N-p-Toluyl-, N-o-Ethylphenyl-, N-m-Ethylphenyl- oder N-p-Ethylphenyl-1,2-ethylendiamin; N,2-Dicyclohexyl-, N,2-Dicyclopentyl-, N,2-Dibenzyl-, N,2-Diphenyl-, N-2-Di-(o-methoxyphenyl)-, N,2-Di-(m-methoxyphenyl)-, N,2-Di-(p-methoxyphenyl)-, N,2-Di-(2,5-dimethylphenyl)-, N,2-Di-(2,6-dimethylphenyl)-, N,2-Di-(2,4-dimethylphenyl)-, N,2-Di-(2,3-Dimethylphenyl)-, N,2-Di-(o-toluyl)-, N,2-Di-(m-toluyl)-, N,2-(p-toluyl)-, N,2-Di-(o-ethylphenyl)-, N,2-Di-(m-ethylphenyl) - oder N,2-Di-p-ethylphenyl)-1,2-ethylendiamin.

Als Nitrile der Formel (III) kommen z.B. in Betracht: Stearinsäurenitril, Palmitinsäurenitril, Essigsäurenitril, Propionsäurenitril, Buttersäurenitril, Isobuttersäurenitril, Pentancarbonsäurenitril, 2-Ethylhexancarbonsäurenitril, Caprylsäurenitril, Trimethylessigsäurenitril, Bernsteinsäurenitril, Malonsäuredinitril, Isovaleriansäurenitril, Valeriansäurenitril, Glutarsäuredinitril, Adipinsäuredinitril, 2,3-Dimethylbenzoesäurenitril, 2,4-Dimethylbenzoesäurenitril, 2,5-Dimethylbenzoesäurenitril, 2,6-Di-methylbenzoesäurenitril, Benzoesäurenitril, Phenylpropionsäurenitril, Cyclopentancarbonsäurenitril, Cyclohexansäurenitril, Phenylessigsäurenitril, o-Toluylsäurenitril, m-Toluylsäurenitril, o-Toluylsäurenitril.

Als Katalysatoren verwendet man Kupfersalze organischer oder anorganischer Säuren, wie Kupfer-I-chlorid, Kupfer-II -chlorid oder Kupfer-II-acetat. Man verwendet sie in Mengen von 0,1 bis 5 Gewichtsteilen, bezogen auf 100 Gewichtsteile Diamin.

Man nimmt die Umsetzung der Diamine mit den Nitrilen in der Flüssigphase und in Gegenwart der Kupfersalze bei Temperaturen von 110 bis 300°C, vorzugsweise 150 bis 200°C, drucklos oder unter Druck, kontinuierlich oder diskontinuierlich vor. In der Regel dient das Reaktionsgemisch auch als Lösungsmedium. Man kann aber auch unter den Reaktionsbedingungen inerte organische Lösungsmittel, wie aliphatische oder aromatische Kohlenwasserstoffe, z.B. Benzol, Toluol, Xylol, Petrolether oder Ligroin verwenden. Die Ausgangsstoffe werden miteinander in stöchiometrischer Menge oder im Überschuß des einen oder anderen Ausgangsstoffs, vorzugsweise in einem Verhältnis von 1 bis 1,1 Mol Ausgangsstoff (III) je Mol Ausgangsstoff (III), umgesetzt.

Die Reaktion wird z.B. durchgeführt, indem man ein Gemisch aus 1,2-Diamin, Nitril und dem Kupfersalz 2 bis 4 Stunden auf der Reaktionstemperatur hält, wobei die Reaktion unter lebhafter Ammoniakentwicklung abläuft. Das als Feststoff ausgeschiedene Reaktionsprodukt kann in üblicher Weise, z.B. durch Destillation oder Umkristallisation, gereinigt werden.

Die nach dem Verfahren der Erfindung herstellbaren 2-Imidazoline der Formel (I) sind wertvolle Ausgangsstoffe für die Herstellung von Farbstoffen, Pflanzenschutzmitteln, Pharmazeutika und Polymeren. Sie werden auch als Katalysatoren für Polymerisationsreaktionen und Aldolkondensationen eingesetzt. Durch Dehydrierung an Aluminium/Zinkoxidkatalysatoren liefern sie die entsprechenden Imidazole.

Die in den folgenden Beispielen aufgeführten Teile bedeuten Gewichtsteile.

**Beispiel 1**

120 Teile Ethylendiamin werden mit 82 Teilen Acetonitril und 2,2 Teilen Cu-II-acetat. $H_2O$ in einem 0,6 l-$V_2$A-Autoklaven auf 200°C erhitzt. Man hält die Temperatur 2 h auf 200°C, wobei sich im Autoklaven ein Druck von 45 bar einstellt. Nach dem Erkalten wird das $NH_3$-Gas über eine Abgasleitung entspannt. Das kristalline Reaktionsprodukt (= 129 Teile) wird aus 250 Teilen Aceton umkristallisiert. Man erhält 118 Teile 2-Methylimidazolin mit einem Schmelzpunkt von 102,5 bis 103,2°C, entsprechend einer Ausbeute von 70,2 % d.Th. Das Produkt hat nach der GC-Analyse eine Reinheit von 99,5 %.

**Beispiel 2**

148 Teile 1,2-Diaminopropan und 110 Teile Propionitril werden mit 2,2 Teilen Kupfer-II-acetat. $H_2O$ in einem 0,6 l-$V_2$A-Autoklaven 2 h auf 200°C erhitzt. Der Druck steigt auf 52 bar an. Nach dem Erkalten (Enddruck = 25 bar) wird das $NH_3$-Gas über eine Abgasleitung entspannt und das Reaktionsprodukt unter Vakuum destilliert. Bei 1 mbar und 98 bis 110°C erhält man 215 Teile 2-Ethyl-4-methylimidazolin, entsprechend einer Ausbeute von 96,0 % d.Th.

**Beispiel 3**

446 Teile 1,2-Diaminopropan, 618 Teile Benzonitril und 4 Teile Kupfer-II-acetat. $H_2O$ werden an einer Destillationskolonne (Ø 25 mm, Länge 100 mm mit 2 mm Glasringen gefüllt) 11 h am Rückfluß erhitzt bis die $NH_3$-Entwicklung beendet ist. Die Sumpftemperatur erreicht 192°C. Die Reaktionsmischung wird anschließend unter Vakuum destilliert. Als Hauptlauf ($Kp_1$ 139 bis 142°C) erhält man 935 Teile 2-Phenyl-4(5)-methylimidazolin, entsprechend einer Ausbeute von 96,6 % d.Th. Das Produkt hat nach der GC-Analyse eine Reinheit von 99,6 %.

**Beispiel 4**

515 Teile Benzonitril werden in einem Rührkolben, der mit Thermometer, Rückflußkühler und Tropftrichter ausgerüstet ist, mit 9 Teilen Kupfer-II-acetat. $H_2O$ auf 180°C (Rückfluß) erhitzt. 300 Teile Ethylendiamin werden in 4 Stunden so zugetropft, daß die Temperatur im Rührkolben nicht unter 180°C abfällt. Man kocht weitere 4 h unter Rückfluß bis die Sumpftemperatur auf 265°C gestiegen ist. Die Reaktionsmischung wird unter Vakuum destilliert. Man erhält bei 0,5 bis 1 mbar und 160 bis 180°C 695 Teile 2-Phenylimidazolin, entsprechend einer Ausbeute von 95,2 % d.Th.

**Beispiel 5**

Es werden, wie in Beispiel 4 beschrieben, 515 Teile Benzonitril mit 10 Teilen Kupfer-II-chlorid unter Rückfluß gekocht. Innerhalb von 6 h werden 300 Teile Ethylendiamin zugetropft. Nach weiteren 2 h wird eine Sumpftemperatur von 265°C erreicht. Die Destillation des Rohprodukts ergibt als Hauptlauf 680 Teile 2-Phenylimidazolin mit einer Reinheit von 97,8 % (GC-Analyse).

**Beispiel 6**

Eine Mischung aus 120 Teilen Ethylendiamin, 108 Teilen Adipinsäuredinitril und 1 Teil Kupfer-II-acetat wird entsprechend Beispiel 3 umgesetzt. Der Endstoff wird aus Aceton umkristallisiert. Man erhält 162 Teile 1,4-Di-(imidazolinyl -2)-butan vom Schmelzpunkt 218°C, entsprechend einer Ausbeute von 83,5 % d.Th.

**Beispiel 7**

265 Teile Stearinsäurenitril, 60 Teile Ethylendiamin und 1 Teil Kupfer-I-chlorid werden 4 Stunden am Rückfluß erhitzt. Die Temperatur steigt von 130°C auf 200°C am Ende der Reaktion an. Das Reaktionsgemisch wird unter Vakuum destilliert. Man erhält 287 Teile 2-Heptadecylimidazolin vom Siedepunkt $Kp_1$ 198 bis 215°C, entsprechend einer Ausbeute von 93,2 % d.Th.

**Beispiel 8**

136 Teile N-Phenylethylendiamin, 41 Teile Acetonitril und 1 Teil Kupfer-II-acetat werden 4 h am Rückfluß erhitzt, dann wird das Reaktionsgemisch unter Vakuum destilliert. Man erhält 125 Teile 1-Phenyl-2-methylimidazolin vom $Kp_1$ 100 bis 102° C, entsprechend einer Ausbeute von 78,1 % d.Th.

**Beispiel 9**

136 Teile N-Phenylethylendiamin, 103 Teile Benzonitril und 1 Teil Kupfer-II-acetat werden entsprechend Beispiel 6 4 h am Rückfluß erhitzt und unter Vakuum destilliert. Man erhält 160 Teile 1,2-Diphenylimidazolin vom Kp 170 bis 172° C (Fp. = 73° C), entsprechend einer Ausbeute von 72,1 % d.Th.

**Beispiel 10**

136 Teile N-Phenylethylendiamin, 109 Teile Hexahydrobenzonitril und 1 Teil Kupfer-II-acetat werden 4 h am Rückfluß erhitzt. Dann wird unter Vakuum destilliert. Man erhält 198 Teile 1-Phenyl-2-cyclohexylimidazolin als hellgelbe Flüssigkeit vom $Kp_1$ 143 bis 145° C, entsprechend einer Ausbeute von 86,8 % d.Th.

**Beispiel 11**

136 Teile N-Phenylethylendiamin, 117 Teile p-Tolunitril und 1 Teil Kupfer-II-acetat werden 4 h am Rückfluß erhitzt. Dann wird unter Vakuum destilliert. Man erhält 182 Teile 1-Phenyl-2-p-tolylimidazolin vom $Kp_1$ 173 bis 175° C (Fp = 52° C) entsprechend einer Ausbeute von 77,1 % d.Th.

**Beispiel 12**

55 Teile N-(2-Methoxyphenyl)-ethylendiamin, 36 Teile Hexahydrobenzonitril und 0,3 Teile Kupfer-II-acetat werden 2 h am Rückfluß erhitzt. Dann wird unter Vakuum destilliert. Man erhält 53 Teile 1-(2-Methoxyphenyl)-2-cyclohexylimidazolin vom $Kp_1$ 170° C (Fp = 75° C), entsprechend einer Ausbeute von 68,5 % d.Th.

**Beispiel 13**

136 Teile N-Phenylethylendiamin, 131 Teile 3,4-Dimethyl-benzonitril und 1 Teil Kupfer-II-acetat werden 4 h am Rückfluß erhitzt. Dann wird das Reaktionsgemisch unter Vakuum destilliert. Man erhält 208 Teile 1-Phenyl-2-(3,4-dimethylphenyl) -imidazolin vom $Kp_1$ 178 bis 180° C (Fp = 90° C), entsprechend einer Ausbeute von 83,2 % d.Th.

**Beispiel 14**

55 Teile N-(2,5-Dimethylphenyl)-ethylendiamin, 34 Teile Benzonitril und 0,3 Teile Kupfer-II-acetat werden 4 h am Rückfluß erhitzt. Dann wird das Reaktionsgemisch destilliert. Man erhält 60 Teile 1-(2,5-Dimethylphenyl)-2-phenylimidazolin vom $Kp_3$ 185° C (Fp = 90° C), entsprechend einer Ausbeute von 72,2 % d.Th.

**Beispiel 15**

74 Teile 1,2-Diaminopropan, 41 Teile Propionitril und 0,5 Teile Kupfer-II-acetat werden 4 h am Rückfluß erhitzt. Dann wird das Reaktionsprodukt unter Vakuum destilliert. Man erhält 80 Teile 2-Ethyl-5-methylimidazolin vom $Kp_{15}$ 110 bis 112° C, entsprechend einer Ausbeute von 81,6 % d.Th.

**Beispiel 16**

74 Teile 1,2-Diaminopropan, 103 Teile Benzonitril und 1 Teil Kupfer-II-acetat werden 4 h am Rückfluß erhitzt. Man destilliert das Reaktionsprodukt unter Vakuum und erhält 108 Teile 2-Phenyl-5-methylimidazolin vom $Kp_{15}$ 180 bis 183°C, entsprechend einer Ausbeute von 67,5 % d.Th.

**Patentansprüche**

1. Verfahren zur Herstellung von 2-Imidazolinen der Formel (I)

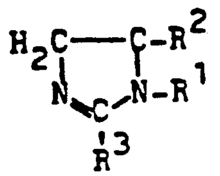

I,

in der $R^1$ und $R^2$ ein Wasserstoffatom, einen Alkylrest mit 1 bis 18 C-Atomen, einen Cyclohexyl- oder Cyclopentylrest, einen Aralkylrest mit 7 bis 12 C-Atomen oder einen Phenyl- oder Naphthylrest, der durch Alkyl- oder Alkoxygruppen mit jeweils 1 bis 4 C-Atomen substituiert sein kann, $R^3$ einen Alkylrest mit 1 bis 18 C-Atomen, einen Cyclohexyl- oder Cyclopentylrest, einen Aralkylrest mit 7 bis 12 C-Atomen, oder einen Phenyl- oder Naphthylrest, der $C_1$-$C_4$ durch Alkyl - oder $C_1$-$C_4$ Alkoxygruppen substituiert sein kann, oder einen Rest der Formel

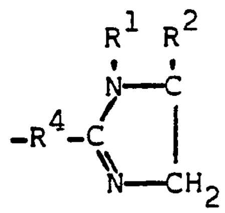

und $R^4$ einen Alkylenrest mit 1 bis 4 C-Atomen bedeuten, durch Umsetzung von 1,2-Diaminen der Formel (II)

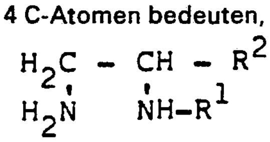

II

mit Nitrilen der Formel (III)
$R^5$ - CH III,
in der $R^5$ die für $R^3$ genannte Bedeutung hat oder für den Rest -$R^4$-CN steht, dadurch gekennzeichnet, daß man die Umsetzung in der Flüssigphase bei Temperaturen von 110 bis 300°C und in Gegenwart von 0,1 bis 5 Gewichtsteilen, bezogen auf 100 Gewichtsteile Diamin, an Kupfersalzen vornimmt.

2. Verfahren nach Anspruch 1, dadurch gekennzeichnet, daß man die Umsetzung bei Temperaturen von 150 bis 200°C vornimmt.

**Claims**

1. A process for the preparation of a 2-imidazoline of the formula (I)

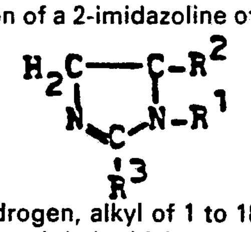

I,

where $R^1$ and $R^2$ are each hydrogen, alkyl of 1 to 18 carbon atoms, cyclohexyl or cyclopentyl, aralkyl of 7 to 12 carbon atoms, or phenyl or naphthyl which may be substituted by alkyl or alkoxy, each of 1 to 4 carbon atoms, $R^3$ is alkyl of 1 to 18 carbon atoms, cyclohexyl or cyclopentyl, aralkyl of 7 to 12 carbon atoms, or phenyl or naphthyl which may be substituted by $C_1$-$C_4$-alkyl or $C_1$-$C_4$-alkoxy, or a radical of the formula

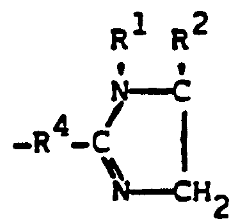

where $R^4$ is alkylene of 1 to 4 carbon atoms, by reacting a 1,2-diamine of the formula (II)

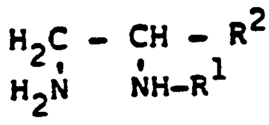

II,

with a nitrile of the formula (III)

$R^5$ - CN III,

where $R^5$ has the same meanings as $R^3$, or is -$R^4$-CN, wherein the reaction is carried out in the liquid phase at a temperature of from 110 to 300°C and in the presence of from 0.1 to 5 parts by weight, based on 100 parts by weight of diamine, of a copper salt.

2. A process as claimed in claim 1, wherein the reaction is carried out at a temperature of from 150 to 200°C.

**Revendications**

Procéde de préparation de 2-imidazolines de formule (I)

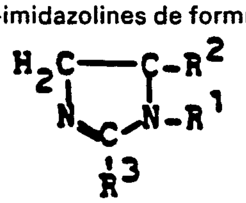

I

dans laquelle $R^1$ et $R^2$ représentent chacun un atome d'hydrogène, un radical alkyle à 1 - 18 atomes de C, un radical cyclohexyle ou cyclopentyle, un radical aralkyle à 7 - 12 atomes de C ou un radical phényle ou naphtyle qui peut être substitué par des groupes alkyle ou alcoxy à 1 - 4 atomes de C, $R^3$ représente un radical alkyle à 1 - 18 atemes de C, un radical cyclohexyle eu cyclopentyle, un radical aralkyle à 7-12 atomes de C ou un radical phényle ou naphtyle qui peut être substitué par des groupes alkyle en $C_1$-$C_4$ ou alcoxy en $C_1$-$C_4$, ou un radical de formule

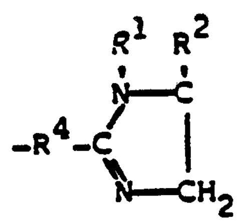

et $R^4$ représente un radical alkylène à 1 - 4 atomes de C, par réaction de 1,2-diamines de formule (II)

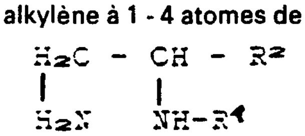

II

avec des nitriles de formule (III)

$R^5$ - CN III

dans laquelle $R^5$ a la signification donnée pour $R^3$ ou est mis pour le radical -$R^4$-CH, caractérisé en ce qu'on procède à la réaction en phase liquide à des températures de 110 à 300°C et en presence de 0,1 à 5 parties en poids, par rapport à 100 parties en poids de diamine, de sels de cuivre.

2 Procédé selon la revendication 1, caractérisé en ce qu'on procède à la réaction à des températures de 150 à 200°C.